# EUROPÄISCHE PATENTSCHRIFT

(11) **EP 3 409 193 B1**
(45) Veröffentlichungstag und Bekanntmachung des Hinweises auf die Patenterteilung: **25.12.2019**
(21) Anmeldenummer: 17174253.9
(22) Anmeldetag: 02.06.2017
(51) Int. Cl.: A61B 5/055, G01R 33/28

(54) **POSITIONIEREN EINER MR-KÖRPERSPULE**
POSITIONING OF AN MR BODY COIL
POSITIONNEMENT D'UNE BOBINE DE CORPS RM

(43) Veröffentlichungstag der Anmeldung: 05.12.2018
(73) Patentinhaber: Siemens Healthcare GmbH, 91052 Erlangen (DE)
(72) Erfinder: Popescu, Stefan, 91056 Erlangen (DE)

(56) Entgegenhaltungen:
- EP-A1- 2 927 854
- WO-A1-99/52094
- WO-A1-2015/169655
- DE-A1-102009 005 188
- DE-A1-102012 209 190
- DE-A1-102015 213 935
- US-A1- 2005 254 714
- US-A1- 2013 165 767
- US-A1- 2014 070 811
- WEIDUSCHAT N ET AL: "Localizing Broca's area for transcranial magnetic stimulation: Comparison of surface distance measurements and stereotaxic positioning", BRAIN STIMULATION, ELSEVIER, AMSTERDAM, NL, Bd. 2, Nr. 2, 1. April 2009 (2009-04-01), Seiten 93-102, XP026044479, ISSN: 1935-861X, DOI: 10.1016/J.BRS.2008.09.005 [gefunden am 2008-12-09]
- ETTINGER G J ET AL: "Experimentation with a transcranial magnetic stimulation system for functional brain mapping", MEDICAL IMAGE ANAL, OXFORDUNIVERSITY PRESS, OXFORD, GB, Bd. 2, Nr. 2, 1. Juni 1998 (1998-06-01), Seiten 133-142, XP009093924, ISSN: 1361-8423

## Beschreibung

Die Erfindung betrifft eine Körperspule zur Verwendung bei einer Kernspintomografie ("MR-Körperspule"). Die Erfindung betrifft auch ein Kernspintomografie-System ("MR-System"), das eine MR-Körperspule und eine Bilderfassungseinrichtung aufweist. Die Erfindung betrifft ferner ein Verfahren zum Betreiben einer MR-Körperspule. Die Erfindung betrifft zudem ein Verfahren zum Positionieren einer MR-Körperspule an einem zu untersuchenden Objekt, bei dem ein Bild der MR-Körperspule von einer Kamera aufgenommen wird und mittels der Aufnahme eine Position der Körperspule bestimmt wird.

Auf dem technischen Gebiet der medizinischen bildgebenden Diagnostik, insbesondere Kernspinresonanz, besteht ein Bedürfnis nach autonom arbeitender Bildgebungsausrüstung, um Bedienfehler zu verhindern und auszuschließen und/oder um auch weniger erfahrene Bediener unter sicherer Einhaltung von Qualitätsrichtlinien durch den Bildgebungsablauf führen zu können. So besteht zum effizienten Betrieb eines Magnetresonanztomographie-Aufnahmegeräts (auch als "MR-Scanner" bezeichnet) eine Anforderung an eine genaue Positionierung von Empfangsspulen, insbesondere Lokalspulen, auf einem zu untersuchenden Körper eines Patienten ("Körperspulen") im Hinblick auf die Anatomie des Körpers als auch auf eine genaue Positionierung der Körperregionen in einem Bereich mit höchster Homogenität des Magnetfelds.

Bisher liegt die korrekte Positionierung der Körperspulen in der Hand des Bedieners. Eine nicht korrekte Positionierung der Körperspulen kann durch menschliche Fehler wie Stress, Ermüdung, usw. und/oder aufgrund schlechter Schulung usw. auftreten. Die nicht korrekte Positionierung führt zu einer Verschlechterung einer Mess- und Bildqualität der MR-Aufnahme und/oder zu einer Notwendigkeit, die Position der Empfangsspulen zu korrigieren und die MR-Aufnahme folgend zu wiederholen. Möglicherweise muss ein Patient erneut einbestellt werden, um die MR-Aufnahme zu wiederholen.

Verfahren zur Positionierung von MR-Spulen und/oder Patienten sind beispielhaft in den Druckschriften US 8212558 B2 und US 7180294 B2 beschrieben. Auch eine korrekte Positionierung des Patienten bzw. seiner zu untersuchenden Organe in dem MR-Scanner beruht auf den Fähigkeiten des Bedieners. Der Bediener sollte in der Lage sein, sichtbare anatomische Merkmale des Körpers zu identifizieren und auszuwählen und dann ein Laserpositionierungssystem zu nutzen, um einen Patiententisch so zu verschieben, dass sich die zu untersuchenden Organe in dem magnetischen Isozentrum des MR-Scanners befinden.

US 2013/0342350 A1 offenbart ein Verfahren, das auf einer Videokamera und Bildverarbeitungsalgorithmen beruht, um automatisch Positionen und Orientierungen von Körperspulen in Bezug auf eine Patientenanatomie zu erfassen und eine akustische und visuelle Rückmeldung an einen Bediener zu liefern, um Positionierungsfehler zu verhindern und zu korrigieren, bevor eine Messaufnahme ("Scan") durchgeführt wird oder sogar noch bevor der Patiententisch in einen Aufnahmeraum des MR-Scanners verschoben wird. Die Genauigkeit dieses Verfahrens hängt von der Zuverlässigkeit der Bildverarbeitungsalgorithmen ab. Dies ist beispielsweise für den Fall nachteilig, dass ein Körper eines Patienten mit einer Decke bedeckt wird, um eine Unterkühlung zu vermeiden. In diesem Fall und wenn die Patientenvorbereitung einschließlich der Platzierung der Spulen und das Verlegen der Decke außerhalb eines Sichtfelds der Videokamera durchgeführt wurde, wird eine spätere Erfassung der Spulenposition erschwert.

US 2013/165767 A1 offenbart MR-Lokalspulen mit auf ihnen zu ihrer Positionierung angebrachten IR-LEDs.

Es ist die **Aufgabe** der vorliegenden Erfindung, die Nachteile des Standes der Technik zumindest teilweise zu überwinden und insbesondere eine Möglichkeit bereitzustellen, um eine Position und/oder eine Orientierung von MR-Körperspulen besser erfassen zu können.

Diese Aufgabe wird gemäß den Merkmalen der unabhängigen Ansprüche gelöst. Bevorzugte Ausführungsformen sind insbesondere den abhängigen Ansprüchen entnehmbar.

Die Aufgabe wird gelöst durch eine MR-Körperspule, an der mehrere Lichtquellen an einer vorgegebenen Position befestigt sind.

Dabei ist eine MR-Körperspule im Sinne einer Lokalspule und/oder Oberflächenspule gemeint und nicht etwa eine Ganzkörperspule, die üblicherweise fest in einem MR-Scanner eingebaut ist.

Diese Körperspule ergibt den Vorteil, dass eine leuchtende Lichtquelle von einem Bilderkennungssystem, insbesondere von einer (Video-)Kamera, besonders deutlich erkennbar ist. Dadurch wiederum sind die Position der Lichtquelle und die entsprechende Stelle der Körperspule besonders genau bestimmbar. Das von der Lichtquelle abgestrahlte Licht kann insbesondere auch durch eine Patientendecke hindurch strahlen bzw. sichtbar sein, so dass eine ausreichend genaue Bestimmung der Position und ggf. einer Ausrichtung der Körperspule mittels einer Bildverarbeitung auch für einen zugedeckten Patienten ermöglicht wird.

Die MR-Körperspule ist mit einer Auflagefläche zur Auflage auf einem zu untersuchenden Objekt und einer der Auflageseite abgewandten Außenseite versehen, wobei an der Außenseite mehrere Lichtquellen an einer vorgegebenen Position befestigt sind. Die Außenseite kann auch einen Rand der Körperspule beinhalten.

Es ist eine Ausgestaltung, dass mindestens eine Lichtquelle mindestens eine Leuchtdiode umfasst. Die Verwendung von Leuchtdioden ist vorteilhaft, weil sie sehr langlebig sind, einen kompakten Aufbau aufweisen, sehr hell leuchten und ein scharfes spektrales Spektrum zeigen.

Es ist noch eine Ausgestaltung, dass die Lichtquellen mehrere in einer vorgegebenen Anordnung angeordnete Lichtquellen aufweisen. Diese Ausgestaltung ergibt den Vorteil, dass durch die Anordnung auch eine Ausrichtung der Körperspule mit bildverarbeitenden Mitteln besonders einfach erkennbar ist. Die Ausrichtung kann eine Drehlage und ggf. auch einen Neigungswinkel der Körperspule umfassen. So können neben einem Versatz auch eine Verdrehung und ggf. eine Verkippung der Körperspule gegenüber einer Sollposition festgestellt werden. Die Sollposition kann in Bezug auf den Patienten und/oder in Bezug auf eine Patientenliege definiert sein. Zusätzlich oder alternativ kann mittels dieser Ausgestaltung bei biegsamen Körperspulen auch deren Biegungsgrad festgestellt werden.

Die Ausrichtung kann auch quantitativ festgestellt werden.

Es ist eine weitere Ausgestaltung, dass mindestens zwei Lichtquellen Licht unterschiedlicher spektraler Zusammensetzung, insbesondere unterschiedlicher Farbe, abstrahlen. Dies ergibt den Vorteil, dass eine Position und Ausrichtung der Körperspule mit bildverarbeitenden Mitteln besonders genau erkennbar ist.

Es ist noch eine weitere Ausgestaltung, dass mindestens eine Lichtquelle eine Infrarot (IR)-Lichtquelle ist. Die Infrarot-Lichtquelle weist den Vorteil auf, dass die davon abgestrahlte Infrarotstrahlung Hindernisse wie eine (z.B. Woll- oder Stoff-)Decke aufgrund von geringeren Streuverlusten besser durchdringen kann als sichtbares Licht. Insbesondere eine Wolldecke kann eine Körpertemperatur effektiv durch Verhindern eines Wärmeverlusts aufgrund von Luftkonvektion halten, während sie für Infrarotstrahlung gut durchlässig ist.

Die Infrarot-Lichtquelle kann insbesondere nur Infrarotstrahlung - und kein sichtbares Licht - abstrahlen. Eine sichtbares Licht abstrahlende Lichtquelle kann auch IR-Licht abstrahlen oder alternativ nur sichtbares Licht abstrahlen.

Die von der Infrarot-Lichtquelle abgestrahlte Infrarotstrahlung kann nahes Infrarot (NIR)-Licht, mittleres Infrarot (MIR)-Licht und/oder fernes Infrarot (FIR)-Licht sein.

Es ist grundsätzlich möglich, dass die mehreren Lichtquellen eine Kombination von Lichtquellen, die sichtbares Licht ausstrahlen, und von Lichtquellen, die nur IR-Strahlung ausstrahlen, umfassen.

Mehrere Lichtquellen sind kodiert blinkend betreibbar. Dies ermöglicht vorteilhafterweise eine Erkennung der Lichtquelle anhand ihres Blink-Codes. Dies ist besonders vorteilhaft, wenn keine Erkennung einer Lichtquelle anhand ihrer Farbe vorgenommen wird, speziell im Zusammenhang mit Infrarot-Lichtquellen. Die Nutzung eines Blink-Codes bzw. eines kodierten Blinkens kann grundsätzlich zusätzlich zu einer Farbkodierung verwendet werden.

Die Lichtquellen sind mit einem individuellen Blink-Code kodiert blinkend betreibbar. Mehrere Lichtquellen können zusätzlich gruppenweise mit dem gleichen Blink-Code kodiert blinkend betreibbar sein. Die individuelle Kodierung ergibt den Vorteil, dass jede Lichtquelle und deren Position an der Körperspule individuell identifizierbar ist.

Der Blink-Code ist ein binärer Code. Der Blink-Code kann ein Morse-Code sein.

Eine Blinkrate oder Blinkfrequenz des Blink-Codes kann so gewählt sein, dass sie von einer Bildaufnahmeeinrichtung sicher detektierbar ist. Dazu sollte die Blinkrate merklich kleiner sein als die Bildaufnahmerate, insbesondere maximal nur halb so groß sein wie die Bildaufnahmerate, um das Nyquist-Shannon-Abtasttheorem zu erfüllen. Es ist eine Weiterbildung, dass die Blinkrate mindestens fünfmal kleiner ist als die Bildaufnahmerate. Nimmt beispielsweise eine Kamera Bilder mit einer Bildaufnahmerate von 25 bis 30 Bildern pro Sekunde (fps) auf, blinkt die Lichtquelle vorteilhafterweise maximal fünfmal pro Sekunde auf.

Es ist auch eine Ausgestaltung, dass mindestens eine Lichtquelle zur Datenübertragung kodiert blinkend betreibbar ist. Dies ergibt den Vorteil, dass auch (über die Identifizierung der Lichtquelle hinausgehende) Information von der Körperspule auf eine Bilderfassungseinrichtung übertragbar ist. Solche Information kann Zustandsinformation über einen Zustand der Körperspule wie eine Statusinformation oder eine Fehlerinformation usw. sein. Die Fehlerinformation kann einen oder mehrere Fehlercodes umfassen, die z.B. mit Fehlerfällen korrespondieren, bei denen elektronische Module der Körperspule defekt sind. Dies ist besonders vorteilhaft, wenn die Körperspule eine drahtlos kommunizierende Körperspule ist und die drahtlose Kommunikation mit dem MR-Scanner unterbrochen wird, z.B. aufgrund von externer Interferenz oder aufgrund eines Fehlers oder Versagens in der Elektronik der Körperspule.

Es ist eine Weiterbildung, dass die mindestens eine Lichtquelle über einen Spulenanschluss der Körperspule mit Strom versorgbar ist. Die mindestens eine Lichtquelle wird einzeln oder gruppenweise mittels eines jeweiligen Treibers mit Strom versorgt.

Eine weitere Ausgestaltung ist ein MR-System, aufweisend eine MR-Körperspule nach einem der vorhergehenden Ansprüche und eine Bilderfassungseinrichtung zum Erfassen von Licht, das von der mindestens einen Lichtquelle der MR-Körperspule abstrahlbar ist. Das MR-System kann analog zu der Körperspule ausgebildet werden und ergibt die gleichen Vorteile.

Die Bilderfassungseinrichtung kann eine Komponente des MR-Scanner darstellen oder eine eigenständige Einrichtung sein. Insbesondere falls die Bilderfassungseinrichtung eine eigenständige Einrichtung ist, ist sie auch unabhängig von dem MR-Scanner betreibbar. Ist die Bilderfassungseinrichtung in den MR-Scanner integriert, kann sie insbesondere vor Einschieben eines Patienten in den Aufnahmeraum des MR-Scanners betrieben werden.

Es ist eine Weiterbildung, dass eine Auswerteeinrichtung zur Auswertung der mit der Bilderfassungseinrichtung ausgenommenen Bilder mit der Bilderfassungseinrichtung verbunden oder in die Bilderfassungseinrichtung integriert ist. Die Auswerteeinrichtung kann anhand der erfassten Aufnahmen oder Bilder die Position und ggf. Ausrichtung und/oder Biegung der Körperspule bestimmen, insbesondere gegen jeweilige Sollwerte. Insbesondere für den Fall, dass die mindestens eine Lichtquelle mehrere in einer vorgegebenen Anordnung angeordnete Lichtquellen aufweist, kann dies mittels einer Mustererkennung durchgeführt werden.

Es ist eine Ausgestaltung, dass die Bilderfassungseinrichtung mindestens eine Kamera und einen Tiefensensor aufweist. Diese Ausgestaltung ermöglicht eine besonders genaue Feststellung einer Position, insbesondere dreidimensionalen Position, und Orientierung der Lichtquellen. Dies ist besonders vorteilhaft bei stark konturierten Körperspulen. Auch wird so eine besonders genaue Bestimmung einer Biegung der Körperspule ermöglicht.

Es ist eine Weiterbildung, dass der Tiefensensor oder Messwerte davon dazu verwendbar sind, einen Such- oder Erfassungsbereich für Lichtquellen, insbesondere Infrarot-Lichtquellen, zu beschränken. Dies ist beispielsweise vorteilhaft, um eine Erfassung von Störquellen, z.B. von einem Boden reflektierte Lichtbündel, zu unterdrücken oder zu vermeiden.

Es ist außerdem eine Ausgestaltung, dass die Bilderfassungseinrichtung eine Kamera aufweist, die für eine sichtbares Lichtspektrum empfindlich ist, und eine Kamera aufweist, die für ein Infrarot-Spektrum empfindlich ist. Dies erleichtert die Erfassung von Lichtquellen erheblich, insbesondere von Lichtquellen, die von einer Stoff- oder Wolldecke abgedeckt sind. Die Erfassung kann beispielsweise durch Vergleich oder Kombination von Ergebnissen einer Bilderkennung im sichtbaren Lichtspektrum mit Ergebnissen einer Bilderkennung im IR-Spektrum verbessert werden.

Die beiden Kameras bzw. deren Funktionen können auch in einer einzigen, multispektral empfindlichen Kamera integriert sein. Die beiden Kameras und der Tiefensensor können in einer einzigen Sensoreinheit integriert sein.

Die Aufgabe wird auch gelöst durch ein Verfahren zum Betreiben einer MR-Körperspule (insbesondere mit einer Auflagefläche zur Auflage auf einem zu untersuchenden Objekt und einer der Auflageseite abgewandten Außenseite), bei welchem Verfahren mehrere (an der Außenseite befestigte) Lichtquellen zur Positionierung der MR-Körperspule Licht abstrahlen. Das Verfahren kann analog zu der MR-Körperspule ausgebildet werden und ergibt die gleichen Vorteile.

Eine weitere Ausgestaltung ist ein Verfahren zum Positionieren einer MR-Körperspule an einem zu untersuchenden Objekt, bei dem mehrere an einer MR-Körperspule befestigte Lichtquellen Licht abstrahlen, das Licht von einer Kamera aufgenommen wird und mittels des von der Kamera aufgenommenen Bilds zumindest eine Position der mindestens einen Lichtquelle bestimmt wird. Das Verfahren kann analog zu der Körperspule und zu dem MR-System ausgebildet werden und ergibt die gleichen Vorteile.

Dass zumindest eine Position der mindestens einen Lichtquelle bestimmt wird, kann umfassen, dass nur die Position bestimmt wird oder kann umfassen, dass die Position und mindestens eine weitere Lageeigenschaft der Körperspule wie deren Orientierung (z.B. ein Drehwinkel und/oder ein Neigungswinkel) und/oder deren Biegung bestimmt werden.

Es ist eine Ausgestaltung, dass eine Abweichung zumindest der bestimmten Position von einem Sollwert oder Sollbereich bestimmt wird. So kann vorteilhafterweise festgestellt werden, ob sich die Körperspule an einer geeigneten oder vorgesehenen Position und ggf. in einer korrekten Ausrichtung und/oder Biegung für eine folgende MR-Aufnahme befindet.

Es ist noch eine Ausgestaltung, dass dann, wenn die Abweichung zu dem Sollwert einen vorgegeben Schwellwert überschreitet, eine Information an einen Bediener ausgegeben wird. Der Bediener kann anhand der Information die Position und/oder Ausrichtung der Körperspule korrigieren. Die Information kann eine akustische oder optische Information sein. Die optische Information kann eine in einem Bildschirm dargestellte Information sein und Korrekturrichtungen und/oder Korrekturlängen umfassen.

Das Verfahren kann auch dazu genutzt werden, um durch von mindestens einer Lichtquelle abgestrahlte Lichtsignale Statusinformation der Körperspule an die Bilderfassungseinrichtung zu übertragen. Mittels der Statusinformation kann das MR-System gesteuert werden, beispielsweise ein MR-Gerät abgeschaltet werden, wenn die Statusinformation einen Fehler der Körperspule anzeigt.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden schematischen Beschreibung von Ausführungsbeispielen, die im Zusammenhang mit den Zeichnungen näher erläutert werden. Dabei können zur Übersichtlichkeit gleiche oder gleichwirkende Elemente mit gleichen Bezugszeichen versehen sein.
- Fig.1: zeigt als Schnittdarstellung in Seitenansicht eine MR-Vorrichtung 1;
- Fig.2: zeigt in Draufsicht eine Patientenliege mit einem Patienten, auf den zwei MR-Körperspulen aufgelegt sind;
- Fig.3: zeigt in Schrägansicht eine weitere MR-Körperspule; und
- Fig.4: zeigt in Schrägansicht noch eine weitere MR-Körperspule.

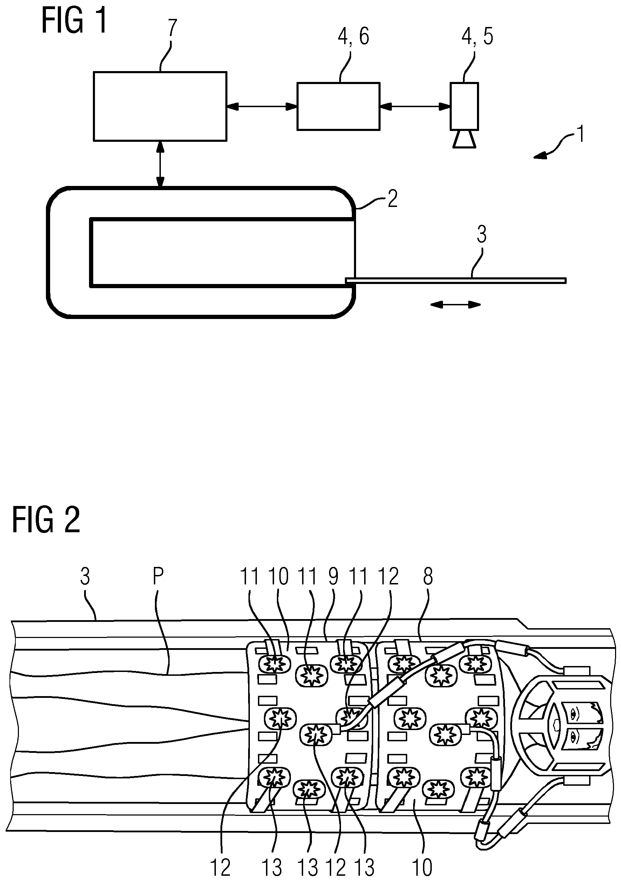

**Fig.1** zeigt eine Kernspinresonanz (MR)-Vorrichtung 1, aufweisend einen MR-Scanner 2 mit einer Patientenliege 3 und mit einer Bilderfassungseinrichtung 4. Die Bilderfassungseinrichtung 4 weist eine Sensoreinrichtung 5 auf, deren Blickfeld von oben auf eine ausgefahrene Patientenliege 3 gerichtet ist. Die Bilderfassungseinrichtung 4 weist ferner eine mit der Sensoreinrichtung 5 gekoppelte Bildauswerteeinrichtung 6 auf. Die Bildauswerteeinrichtung 6 kann wiederum mit einer Steuereinrichtung 7 der MR-Vorrichtung 1 gekoppelt sein.

Die Sensoreinrichtung 5 kann eine einfache RGB-Videokamera sein oder kann eine Sensoreinrichtung mit einer RGB-Videokamera, einer IR-Kamera und/oder einem Tiefensensor (o. Abb.) aufweisen.

**Fig.2** zeigt in Draufsicht die Patientenliege 3. Auf der Patientenliege 3 liegt ein Patient P, auf den zwei gleichartige MR-Körperspulen 8, 9 mit einer jeweiligen Auflagefläche (o. Abb.) aufgelegt sind. An einer der Auflageseite abgewandten Außenseite 10 sind pro MR-Körperspulen 8, 9 jeweils neun Lichtquellen in Form von LEDs 11, 12, 13 an einer jeweils fest vorgegebenen Position und damit auch in einer vorgegebenen Anordnung zueinander befestigt. Die LEDs 11 bis 13 können Komponenten jeweiliger LED-Module sein. Die LED-Module können auch einen Treiber, eine Schirmung usw. aufweisen. Die LED-Module können den elektronischen Modulen entsprechen, z.B. wenn den elektronischen Modulen mindestens eine LED hinzugefügt worden ist.

Die LEDs 11 bis 13 sind in drei parallelen Reihen mit jeweils drei LEDs 11, 12 bzw. 13 angeordnet, die hier dazu vorgesehen sind, entlang einer Längserstreckung des Patienten P ausgerichtet zu sein. In den Reihen ist jeweils die mittlere der LEDs 11 bis 13 seitlich versetzt. Die LEDs 11 bis 13 strahlen Licht unterschiedlicher Farbe aus. So können die LEDs 11 z.B. rotes Licht abstrahlen, die LEDs 12 z.B. grünes Licht abstrahlen und die LEDs 13 z.B. blaues oder gelbes Licht abstrahlen. Also strahlen die Reihen Licht unterschiedlicher Farbe aus. Alternativ können z.B. auch alle LEDs Licht unterschiedlicher Farbe abstrahlen.

Die Kamera 5 ist dazu eingerichtet, das von den LEDs 11 bis 13 abgestrahlte Licht zu detektieren, insbesondere in einem Bild. Das aufgenommene mindestens eine Bild kann mittels der Bildauswerteeinrichtung 6 ausgewertet werden. Weist die Sensoreinrichtung 5 einen Tiefensensor auf, kann dieser zusätzlich zur Bildauswertung herangezogen werden.

Als ein Ergebnis der Bildauswertung können die Positionen der LEDs 11 bis 13 und damit auch der MR-Körperspulen 8, 9 sowie optional deren Ausrichtung und ggf. deren Biegung bestimmt werden, insbesondere in Bezug auf jeweilige Sollwerte. Diese Abweichungen können über die Steuereinrichtung 7 auf einem Monitor (o. Abb.) angezeigt werden. Folglich kann ein den Monitor betrachtender Bediener (o. Abb.) die Positionen usw. der MR-Körperspulen 8, 9 so lange anpassen, bis die Positionen usw. innerhalb der Sollwerte liegen und somit korrekt positioniert sind.

Zusätzlich sind die LEDs 11 bis 13 kodiert blinkend betreibbar, z.B. indem sie mit einem individuellen Morse-Code blinken. Dies verbessert eine Identifizierung der LEDs 11 bis 13 weiter. Insbesondere können so auch LEDs 11, 12, bzw. 13 gleicher Farbe individuell identifiziert werden. Alternativ können LEDs 11, 12, bzw. 13 gleicher Farbe mittels des gleichen Codes blinken.

Zusätzlich oder alternativ zu den LEDs 11 bis 13 können IR-LEDs an den MR-Körperspulen 8, 9 vorhanden sein und mittels der Sensoreinrichtung 5 detektiert werden. In einer Variante können anstelle der LEDs 11 bis 13 identische IR-LEDs verwendet werden. Um die IR-LEDs durch gruppenweise oder individuell identifizieren zu können, werden diese entsprechend kodiert blinkend betrieben.

Die Sensoreinrichtung 5 kann beispielsweise Bilder mit einer Bildaufnahmerate von 25 bis 30 fps aufnehmen, während die Blinkrate bei 5 Hz liegt.

Es ist grundsätzlich auch möglich, mindestens eine der LEDs 11 bis 13 oder mindestens eine der IR-LEDs zur Datenübertragung kodiert blinkend zu betreiben.

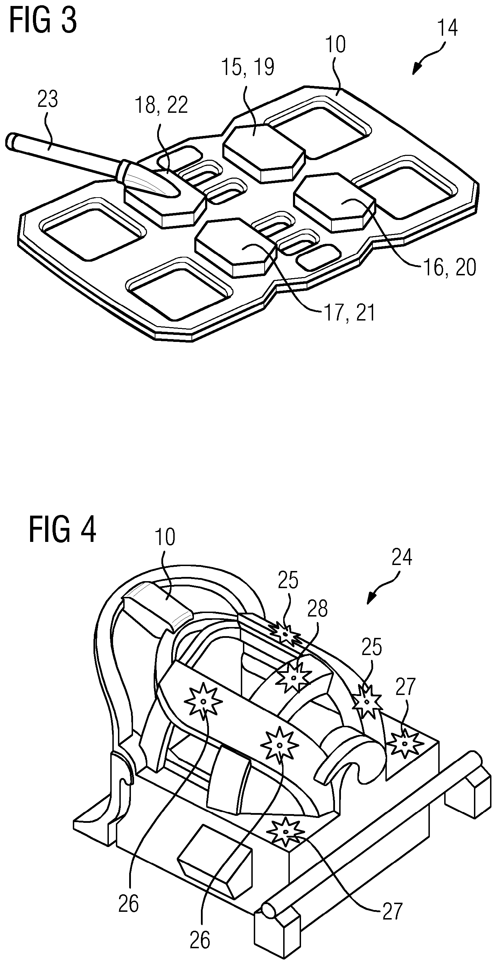

**Fig.3** zeigt in Schrägansicht eine weitere MR-Körperspule 14. Die MR-Körperspule 14 weist an ihrer Außenseite vier LED-Module 15 bis 18 auf, die jeweils mindestens eine LED 19 bis 22 und einen Treiber (o. Abb.) aufweisen. Das LED-Modul 18 weist einen Stromanschluss 23 auf, über den die LED-Module 15 bis 18 mit Strom versorgbar sind. Die LED-Module 15 bis 18 können auch eine Schirmung zum Schutz gegen das in dem MR-Scanner 2 erzeugte Magnetfeld aufweisen.

Die LEDs 19 bis 22 blinken unterschiedlich kodiert und können Licht unterschiedlicher Farbe abstrahlen, um ihre individuelle Identifizierung zu ermöglichen. Insbesondere falls die LEDs 19 bis 22 IR-LEDs sind, blinken sie unterschiedlich kodiert.

**Fig.4** zeigt in Schrägansicht noch eine weitere, nicht unter die Ansprüche fallende MR-Körperspule 24. Diese MR-Körperspule 24 ist zur Aufnahme eines Kopfs des Patienten P vorgesehen und kann auch als Kopfspule bezeichnet werden. An der Außenseite 10 der MR-Körperspule 24 sind LEDs 25 bis 28 vorhanden. Die LEDs 25 bis 27 strahlen paarweise Licht gleicher Farbe und/oder mit gleichem Blink-Code ab, wobei die Farbe und/oder der Blink-Code für unterschiedliche Paare unterschiedlich ist. Eine zentrale LED 28 strahlt Licht nochmals unterschiedlicher Farbe und/oder mit nochmals unterschiedlichem Blink-Code ab.

Allgemein kann unter "ein", "eine" usw. eine Einzahl oder eine Mehrzahl verstanden werden, insbesondere im Sinne von "mindestens ein" oder "ein oder mehrere" usw., solange dies nicht explizit ausgeschlossen ist, z.B. durch den Ausdruck "genau ein" usw.

Auch kann eine Zahlenangabe genau die angegebene Zahl als auch einen üblichen Toleranzbereich umfassen, solange dies nicht explizit ausgeschlossen ist.

## Patentansprüche

1. MR-Körperspule (8, 9; 14; 24) mit einer Auflagefläche zur Auflage auf einem zu untersuchenden Objekt (P) und einer der Auflageseite abgewandten Außenseite (10), wobei an der Außenseite (10) mehrere in einer vorgegebenen Anordnung angeordnete Lichtquellen (11-13; 19-22; 25-28), die insbesondere mehrere Leuchtdioden umfassen, an einer jeweils vorgegebenen Position befestigt sind, **dadurch gekennzeichnet, dass** die MR-Körperspule (8, 9; 14; 24) mindestens einen Treiber aufweist, der dazu eingerichtet ist, die Lichtquellen (11-13; 19-22; 25-28) mittels eines jeweiligen, sie individuell identifizierenden binären Blink-Codes kodiert blinkend zu betreiben.

2. MR-Körperspule (8, 9; 14; 24) nach Anspruch 1, wobei der mindestens eine Treiber dazu eingerichtet ist, mehrere Lichtquellen 11-13; 19-22; 25-28) zusätzlich gruppenweise mit dem gleichen Blink-Code kodiert blinkend zu betreiben.

3. MR-Körperspule (8, 9; 14; 24) nach einem der vorhergehenden Ansprüche, wobei der Blink-Code ein Morse-Code ist.

4. MR-Körperspule (8, 9; 14; 24) nach einem der vorhergehenden Ansprüche, wobei der mindestens eine Treiber dazu dazu eingerichtet ist, die Lichtquellen (11-13; 19-22; 25-28) zur Datenübertragung kodiert blinkend zu betreiben.

5. MR-Körperspule (8, 9; 14; 24) nach Anspruch 4, wobei der mindestens eine Treiber dazu eingerichtet ist, die Lichtquellen (11-13; 19-22; 25-28) zur Übertragung von Zustandsinformation oder Fehlerinformation über einen Zustand der MR-Körperspule (8, 9; 14; 24) kodiert blinkend zu betreiben.

6. MR-Körperspule (8, 9; 14; 24) nach Anspruch 5, wobei die Fehlerinformation einen oder mehrere Fehlercodes umfasst, die mit Fehlerfällen korrespondieren, bei denen elektronische Module der MR-Körperspule (8, 9; 14; 24) defekt sind.

7. MR-Körperspule (8, 9; 14; 24) nach einem der vorhergehenden Ansprüche, wobei mindestens zwei Lichtquellen (11-13; 19-22; 25-28) Licht unterschiedlicher Farbe abstrahlen.

8. MR-Körperspule (8, 9; 14; 24) nach einem der vorhergehenden Ansprüche, wobei mindestens eine Lichtquelle (11-13; 19-22; 25-28) eine Infrarot-Lichtquelle ist.

9. MR-System (1, 8, 9; 14; 24), aufweisend mindestens eine MR-Körperspule und eine Bilderfassungseinrichtung (4) zum Erfassen von Licht, das von der mindestens einen Lichtquelle (11-13; 19-22; 25-28) der mindestens einen MR-Körperspule abstrahlbar ist, **dadurch gekennzeichnet, dass** die mindestens eine MR-Körperspule mindestens eine MR-Körperspule (8, 9; 14; 24) nach einem der vorhergehenden Ansprüche ist.

10. MR-System (1, 8, 9; 14; 24) nach Anspruch 9, wobei die Bilderfassungseinrichtung (4) mindestens eine Kamera und einen Tiefensensor aufweist.

11. Verfahren zum Betreiben einer MR-Körperspule (8, 9; 14; 24), bei dem mehrere in einer vorgegebenen Anordnung angeordnete Lichtquellen (11-13; 19-22; 25-28) der MR-Körperspule (8, 9; 14; 24) zur Positionierung der MR-Körperspule (8, 9; 14; 24) Licht abstrahlen, **dadurch gekennzeichnet, dass** die Lichtquellen (11-13; 19-22; 25-28) zu ihrer Identifizierung mit einem sie individuell identifizierenden binären Blink-Code kodiert blinkend betrieben werden.

12. Verfahren nach Anspruch 11, wobei mindestens eine Lichtquelle (11-13; 19-22; 25-28) zur Datenübertragung kodiert blinkend betrieben wird.

13. Verfahren nach einem der Ansprüche 11 bis 12, bei dem das Licht der Lichtquellen (11-13; 19-22; 25-28) von einer Bilderfassungseinrichtung (4), die mindestens eine Kamera umfasst, aufgenommen wird,
mittels der Aufnahme anhand des von den Lichtquellen (11-13; 19-22; 25-28) jeweils erzeugten Blink-Codes zumindest eine Position der Lichtquellen (11-13; 19-22; 25-28) bestimmt wird,
eine Abweichung zumindest der bestimmten Position von einem Sollwert oder Sollbereich bestimmt wird und
dann, wenn die Abweichung zu dem Sollwert einen vorgegeben Schwellwert überschreitet, eine Information, insbesondere an einen Bediener, ausgegeben wird.

14. Verfahren nach Anspruch 13, wobei die Bilderfassungseinrichtung einen Tiefensensor aufweist, der einen Erfassungsbereich der mindestens einen Kamera zur Aufnahme des abstrahlten Lichts der mindestens einen Lichtquelle (11-13; 19-22; 25-28) einschränkt.

## Claims

1. MR body coil (8, 9; 14; 24) having a supporting surface for support on an object (P) to be examined and an outer side (10) remote from the support side, wherein a plurality of light sources (11-13; 19-22; 25-28) disposed in a predefined arrangement, which in particular comprise a plurality of light-emitting diodes, are fastened to the outer side (10) at a predefined position in each case, **characterised in that** the MR body coil (8, 9; 14; 24) has at least one driver, which is adapted to operate the light sources (11-13; 19-22; 25-28) to flash in a coded manner by means of a respective binary blink code identifying them in an individual manner.

2. MR body coil (8, 9; 14; 24) according to claim 1, wherein the at least one driver is additionally adapted to operate a plurality of light sources (11-13; 19-22; 25-28) to flash in a coded manner in groups with the same blink code.

3. MR body coil (8, 9; 14; 24) according to one of the preceding claims, wherein the blink code is a Morse code.

4. MR body coil (8, 9; 14; 24) according to one of the preceding claims, wherein the at least one driver is adapted to operate the light sources (11-13; 19-22; 25-28) to flash in a coded manner for data transfer.

5. MR body coil (8, 9; 14; 24) according to claim 4, wherein the at least one driver is adapted to operate the light sources (11-13; 19-22; 25-28) to flash in a coded manner for transferring status information or error information regarding the status of the MR body coil (8, 9; 14; 24).

6. MR body coil (8, 9; 14; 24) according to claim 5, wherein the error information comprises one or more error codes, which correspond to cases of error in which electronic modules of the MR body coil (8, 9; 14; 24) are defective.

7. MR body coil (8, 9; 14; 24) according to one of the preceding claims, wherein at least two light sources (11-13; 19-22; 25-28) radiate light of a different colour.

8. MR body coil (8, 9; 14; 24) according to one of the preceding claims, wherein at least one light source (11-13; 19-22; 25-28) is an infrared light source.

9. MR system (1, 8, 9; 14; 24), having at least one MR body coil and an image acquisition device (4) for acquiring light, which can be radiated by the at least one light source (11-13; 19-22; 25-28) of the at least one MR body coil, **characterised in that** the at least one MR body coil is at least one MR body coil (8, 9; 14; 24) according to one of the preceding claims.

10. MR system (1, 8, 9; 14; 24) according to claim 9, wherein the image acquisition device (4) has at least one camera and a depth sensor.

11. Method for operating an MR body coil (8, 9; 14; 24),
wherein a plurality of light sources (11-13; 19-22; 25-28), disposed in a predefined arrangement, of the MR body coil (8, 9; 14; 24) radiate light for positioning the MR body coil (8, 9; 14; 24), **characterised in that** the light sources (11-13; 19-22; 25-28), for the identification thereof, are operated to flash in a coded manner with a binary blink code which identifies them in an individual manner.

12. Method according to claim 11,
wherein at least one light source (11-13; 19-22; 25-28) flashes in a coded manner for data transfer.

13. Method according to one of claims 11 to 12, in which the light of the light sources (11-13; 19-22; 25-28) is acquired by an image acquisition device (4), which comprises at least one camera,
at least one position of the light sources (11-13; 19-22; 25-28) is determined by means of the acquisition on the basis of the blink code generated by the light sources (11-13; 19-22; 25-28) in each case,
a deviation of at least the determined position from a desired value or desired range is determined and
if the deviation from the desired value exceeds a predefined threshold value, information is output, in particular to an operator.

14. Method according to claim 13,
wherein the image acquisition device has a depth sensor, which limits an acquisition region of the at least one camera to acquisition of the radiated light of the at least one light source (11-13; 19-22; 25-28).

## Revendications

1. Bobine (8, 9 ; 14 ; 24) de corps RM, comprenant une surface d'application pour s'appliquer à un objet (P) à examiner et un côté (10) extérieur, loin du côté d'application, dans laquelle, sur le côté (10) extérieur, sont fixées, en une position, respectivement, donnée à l'avance, plusieurs sources (11 à 13 ; 19 à 22 ; 25 à 28) lumineuses, qui comprennent notamment plusieurs diodes électroluminescentes, disposées suivant un agencement donné à l'avance, **caractérisée en ce que** la bobine (8, 9 ; 14 ; 24) de corps RM a au moins un excitateur, conçu pour faire fonctionner en clignotement codé les sources (11 à 13 ; 19 à 22 ; 25 à 28) lumineuses au moyen d'un code de clignotement binaire respectif les identifiant individuellement.

2. Bobine (8, 9 ; 14 ; 24) de corps RM suivant la revendication 1, dans laquelle le au moins un excitateur est conçu pour faire fonctionner en clignotement codé plusieurs sources (11 à 13 ; 19 à 22 ; 25 à 28) lumineuses supplémentairement, groupe par groupe ayant le même code de clignotement.

3. Bobine (8, 9 ; 14 ; 24) de corps RM suivant l'une des revendications précédentes, dans lequel le code de clignotement est un code Morse.

4. Bobine (8, 9 ; 14 ; 24) de corps RM suivant l'une des revendications précédentes, dans laquelle le au moins un excitateur est conçu pour faire fonctionner en clignotement codé les sources (11 à 13 ; 19 à 22 ; 25 à 28) lumineuses en vue de transmettre des données.

5. Bobine (8, 9 ; 14 ; 24) de corps RM suivant la revendication 4, dans laquelle au moins un excitateur est conçu pour faire fonctionner en clignotement codé des sources (11 à 13 ; 19 à 22 ; 25 à 28) lumineuse, afin de transmettre de l'information d'état ou de l'information de défaut sur un état de la bobine (8, 9 ; 14 ; 24) de corps RM.

6. Bobine (8, 9 ; 14 ; 24) de corps RM suivant la revendication 5, dans laquelle l'information de défaut comprend un ou plusieurs codes de défaut, qui correspondent à des cas de défaut dans lesquels les modules électroniques de la bobine (8, 9 ; 14 ; 24) de corps RM sont défectueux.

7. Bobine (8, 9 ; 14 ; 24) de corps RM suivant l'une des revendications précédentes, dans laquelle au moins deux sources (11 à 13 ; 19 à 22 ; 25 à 28) lumineuses émettent de la lumière de couleur différente.

8. Bobine (8, 9 ; 14 ; 24) de corps RM suivant l'une des revendications précédentes, dans laquelle au moins une source (11 à 13 ; 19 à 22 ; 25 à 28) lumineuse est une source de lumière infrarouge.

9. Système (1, 8, 9 ; 14 ; 24) RM suivant ayant au moins une bobine de corps RM et un dispositif (4) de détection d'image pour détecter de la lumière, qui peut être émise par la au moins une source (11 à 13 ; 19 à 22 ; 25 à 28) lumineuse de la au moins une bobine de corps RM, **caractérisé en ce que** la au moins une bobine de corps RM est au moins une bobine (8, 9 ; 14 ; 24) de corps RM suivant l'une des revendications précédentes.

10. Système (1, 8, 9 ; 14 ; 24) RM suivant la revendication 9, dans lequel le dispositif (4) de détection d'image a au moins une caméra et un capteur de profondeur.

11. Procédé pour faire fonctionner une bobine (8, 9 ; 14 ; 24) de corps RM, dans lequel plusieurs sources (11 à 13 ; 19 à 22 ; 25 à 28) lumineuses, disposées suivant un agencement donné à l'avance, de la bobine (8, 9 ; 14 ; 24) de corps RM émettent de la lumière pour la mise en position de la bobine (8, 9 ; 14 ; 24) de corps RM, **caractérisé en ce que** l'on fait fonctionner les sources (11 à 13 ; 19 à 22 ; 25 à 28) lumineuses pour leur identification en clignotement codé avec un code de clignotement binaire les identifiant individuellement.

12. Procédé suivant la revendication 11, dans lequel on fait fonctionnement en clignotement codé au moins une source (11 à 13 ; 19 à 22 ; 25 à 28) lumineuse pour transmettre des données.

13. Procédé suivant l'une des revendications 11 à 12, dans lequel on enregistre la lumière des sources (11 à 13 ; 19 à 22 ; 25 à 28) lumineuses par un dispositif (4) de détection d'image, qui comprend au moins une caméra,
au moyen de l'enregistrement on détermine, à l'aide du code de clignotement produit, respectivement, par les sources (11 à 13 ; 19 à 22 ; 25 à 28) lumineuses, au moins une position des sources (11 à 13 ; 19 à 22 ; 25 à 28) lumineuses,
on détermine un écart d'au moins la position déterminée à une valeur de consigne ou à une région de consigne et, ensuite, si l'écart à la valeur de consigne dépasse une valeur de seuil donnée à l'avance, on émet une information, notamment à un opérateur.

14. Procédé suivant la revendication 11,
dans lequel le dispositif de détection d'image a un capteur de profondeur, qui délimite une région de détection de la au moins une caméra pour l'enregistrement de la lumière émise de la au moins une source (11 à 13 ; 19 à 22 ; 25 à 28) lumineuse.
